# EUROPÄISCHE PATENTSCHRIFT

(11) **EP 1 217 987 B1**
(45) Veröffentlichungstag und Bekanntmachung des Hinweises auf die Patenterteilung: **22.12.2004**
(21) Anmeldenummer: 00962531.0
(22) Anmeldetag: 26.09.2000
(51) Int. Cl.: A61K 7/48

(54) **FILMBILDENDES HAUTREINIGUNGSMITTEL**
FILM-FORMING SKIN CLEANSING AGENT
PRODUITS DE NETTOYAGE DE LA PEAU FILMOGENE

(30) Priorität: 05.10.1999 DE 19947684
(43) Veröffentlichungstag der Anmeldung: 03.07.2002
(73) Patentinhaber: Henkel Kommanditgesellschaft auf Aktien, 40589 Düsseldorf (DE)
(72) Erfinder: LAPENA, Margarita, E-08026 Barcelona (ES); WADLE, Armin, 40699 Erkrath (DE); HEIDE, Barbara, 47809 Krefeld (DE); PUJOL GALIANO, Miracle, E-08329 Teia (ES); DE MORAGAS, Maria, E-08310 Argentona Barcelona (ES)
(86) Internationale Anmeldenummer: PCT/EP2000/009388
(87) Internationale Veröffentlichungsnummer: WO 2001/024770

(56) Entgegenhaltungen:
- WO-A-99/02129
- US-A- 5 939 093
- PATENT ABSTRACTS OF JAPAN vol. 1999, no. 11, 30. September 1999 (1999-09-30) & JP 11 171726 A (JO COSMETICS KK), 29. Juni 1999 (1999-06-29)
- DATABASE WPI Section Ch, Week 197919 Derwent Publications Ltd., London, GB; Class A14, AN 1979-35854B XP002152688 & JP 54 040810 A (LION FAT & OIL CO LTD), 31. März 1979 (1979-03-31)

## Beschreibung

Die Erfindung betrifft ein Hautreinigungsmittel in Form einer fließfähigen oder auf der Haut verteilbaren Zubereitung, die beim Antrocknen auf der Haut einen abziehbaren Film bildet, der beim Ablösen von der Haut Schmutz, Fett, abgestorbene Hautzellen und Mitesser an sich bindet und auf diese Weise die Haut reinigt.

Es ist bekannt, Zubereitungen, die auf der Haut antrocknen und dabei einen festen Rückstand hinterlassen, zur Hautreinigung ohne Wasser einzusetzen. So sind z. B. die sogenannten wasserlosen Handreiniger aus wässrig-organischen Lösungen von Tensiden und Polymeren aufgebaut, die nach dem Einreiben der Hände und dem Verdunsten des Lösungsmittels sich ohne Anwendung von Wasser zusammen mit dem Schmutz mit einem Tuch abwischen - oder in Form von Konkrementen - abrubbeln lassen.

Zur Gesichtsreinigung sind sogenannte Peel-Off Filmmasken bekannt, die ebenfalls aus wässig-alkoholischen Lösungen oder Dispersionen von Polymeren bestehen und sich nach dem Antrocknen als Film von der Haut ablösen lassen.

In Parfums, Cosmetiques, Aromes No. 72 (Dec. 1986), Seite 63 ist z. B. eine abziehbare Gesichtsmaske beschrieben, die 25 Gew.-% einer Latexemulsion und 10 Gew.-% Methylcellulose in einer wässrigen Zubereitung enthält.

Aus Karlheinz Schrader: Grundlagen und Rezepturen der Kosmetika (Hüthig Buch Verlag Heidelberg) 2. Auflage (1989), Seite 581 ist eine Filmmaske mit 10 Gew.-% Polyvinylalkohol in ca. 35 Gew.%igem wässrigem Äthylalkohol bekannt.

Aus EP 711 145 B1 schließlich sind abziehbare Masken bekannt, die eine Kombination aus Polyvinylalkoholpolymeren und hydrophob modifizierten Acrylat- oder Methacrylatpolymeren enthalten. In dieser Druckschrift werden die Probleme einer zu geringen Haftung an der Haut und einer zu langen Trocknungszeit beschrieben, die durch zahlreiche Polymere und Polymerkombinationen verursacht werden.

JP 11171726 A offenbart ein Hautreinigungsmittel in Form eines Pflasters, das ein Polyvinylpyrrolidon und einen Polyvinylalkohol enthält, der zu mindestens 80 % verseift ist, das heißt einen Teilacetylierungsgrad von maximal 20 Mol-% aufweist.

JP 54040810 A offenbart ein Hautreinigungsmittel, das Polyvinylalkohol, Polyvinylpyrrolidon und ein tensidisches EO-PO-Blockcopolymer enthält. Die Gegenwart eines ionischen Polymers ist nicht offenbart.

US 5,939,093 offenbart ein Hautreinigungsmittel in Form eines Pflasters, das 33 Gew.-% der gelösten, filmbildenden und adhäsiven Polymere Polyvinylpyrrolidon, Polyvinylalkohol und Polyacrylsäure sowie 10 Gew.-% eines wasserlöslichen kationischen Copolymers in einem wässrig-alkoholischen Träger enthält.

WO 99/02129 A offenbart Hautreinigungsmittel, die ein Polymer wie Polyvinylalkohol, Polyvinylpyrrolidon oder Polyacrylsäure enthalten und nicht als zusammenhängender Film abgezogen, sondern stückchenweise von der Haut abgerieben werden.

Die bisher bekannten Filmmasken weisen aber noch zahlreiche Mängel auf, z. B. daß sie sich aufgrund mangelhafter Filmstabilität oder zu hoher Haftung nur unvollständig von der Haut abziehen lassen - oder daß die Zubereitung selbst nur eine mangelhafte Lagerstabilität aufweist. Bezüglich der Klebewirkung ist ein optimaler Kompromiß zwischen einer zu starken Hauthaftung und einer geringen Peeling-Wirkung zu finden. Die vorliegende Erfindung hat sich die Aufgabe gestellt, eine für den praktischen Gebrauch optimale Lösung zu finden, mit der die genannten Probleme weitgehend überwunden werden.

Gegenstand der Erfindung ist ein Hautreinigungsmittel in Form einer fließfähigen oder auf der Haut verstreichbaren Zubereitung, die beim Antrocknen auf der Haut einen abziehbaren Film bildet, enthaltend gelöste, filmbildende und adhäsive Polymere in einem wässrig-alkoholischen Träger, wobei als filmbildendes Polymer ein teilacetylierter Polyvinylalkohol mit 10 - 40 Mol% Vinylacetat-Gruppen als adhäsives Polymer ein Polyvinylpyrrolidon und 0,1 -10 Gew.-% eines ionischen Polymers in Form eines wasserlöslichen Salzes ausgewählt gemäß Anspruch 1 enthalten ist.

Als fließfähig oder auf der Haut verteilbar werden dabei sowohl flüssige als auch gelförmige oder pastöse Zubereitungen verstanden, die bei einer Körpertemperatur von ca. 36° C so weich sind, daß sie mit dem Finger oder mit Hilfe eines Applikators auf der Haut verteilt werden können. Geeignete Applikatoren sind z. B. Pinsel oder Rollkugel-(sog. Roll-On) Applikatoren.

Von besonderer Bedeutung für die erfindungsgemäße Anwendung der Hautreinigungsmittel ist die Art und Menge der in dem Mittel enthaltenen polymeren Filmbildner. Eine durch die Kombination aus einem gelösten filmbildenden und wenigstens einem adhäsiven Polymerisat in einer Menge von 10 - 30 Gew.-% in dem Trägermedium wurde der für eine gute Haftung, eine hohe Filmstabilität und eine leichte Ablösbarkeit des Reinigungsfilms von der Haut erforderliche Kompromiß in besonders vorteilhafter Weise gefunden.

Zusätzlich wurde gefunden, daß die Haftung und Reinigungswirkung des Polymerfilms noch verbessert werden kann, wenn zusätzlich ein ionisches Polymer in einer Menge von 0,1 - 10 Gew.%, vorzugsweise 1 - 10 Gew.-% in dem Mittel enthalten ist.

Polyvinylalkohol (PVAL) wird durch Verseifung bzw. Alkoholyse aus Polyvinylacetat hergestellt. Dabei lassen sich teilacetylierte Produkte mit unterschiedlichem Gehalt an Vinyl-acetat-Gruppen isolieren.

Ein geeigneter alkohollöslicher, teilacetylierter PVAL weist bevorzugt einen Gehalt von ca. 12 Mol% Vinylacetatgruppen auf und ist im Handel z. B. unter dem Warenzeichen Mowiol® 18 - 88 (Clariant) erhältlich. In einer bevorzugten Ausführungsform enthält das erfindungsgemäße Hautreinigungsmittel einen teilacetylierten PVAL in einer Menge von 2 - 10 Gew.-%; besonders bevorzugt ist ein Gehalt von 2 - 8 Gew.-%.

Ein erfindungsgemäß geeignetes Polyvinylpyrrolidon (PVP) weist ein mittleres Molekulargewicht von wenigstens 40.000 D, bevorzugt von 700.000 bis 1.000.000 D, auf und ist in einer Menge von 1 - 20 Gew.-%, bevorzugt 8 - 16 Gew.-% , in dem Hautreinigungsmittel enthalten. Solche Produkte sind im Handel unter dem Warenzeichen Luviskol® K (BASF) erhältlich. Ein besonders geeignetes Handelsprodukt ist Luviskol® K80. In einer weiteren bevorzugten Ausführungsform der Erfindung können auch Gemische von Polyvinylpyrrolidonen unterschiedlicher Molekulargewichte verwendet werden deren, mittleres Molekulargewicht im angegebenen Bereich liegt.

Besonders bevorzugt als Filmbildner-Kombination im Sinne der Erfindung ist ein Gehalt von PVP und teilacetyliertem PVAL, z. B. eine Kombination von Luviskol® K 80 und Mowiol® 18 - 88, im Gewichtsverhältnis 2 : 1 bis 5 : 1.

Neben den genannten gelösten Polymeren können auch andere nichtionische, wasserlösliche Polymere wie z. B. Polyacrylamide oder Polyethylenglycole in geringem Umfang, z. B. in Mengen bis 5 Gew.-% enthalten sein.

Weiterhin hat es sich als vorteilhaft zur Verbesserung der Haftwirkung des Polymerfilms und zur Erhöhung der Reinigungsleistung erwiesen, wenn in dem Hautreinigungsmittel zusätzlich ein ionisches Polymer in einer Menge von 0,1 - 10 Gew.-% enthalten ist. Als ionisches Polymer sind entweder anionische oder kationische oder auch zwitterionische Polymere enthalten synthetische, anionische Polymere sind die amionische Homo- oder Copolymerisate der Acrylsäure, Methacrylsäure, Crotonsäure, Itaconsäure, Maleinsäure, Fumarsäure, der 2-Acrylamido-2-methylpropan-sulfonsäure oder der Styrolsulfonsäure. Besonders bevorzugt sind als anionische Polymere Copolymere der Acryloder Methacrylsäure mit nichtionischen Monomeren aus der Gruppe der Acrylsäure (C₁ - C₄)alkylester geeignet. Als nichtionische Comonomere eignen sich z. B. Ethylacrylat, teri.-Butylacrylat oder Methylmethacrylat Diese anionischen Polymere liegen in den erfindungsgemäßen Hautpflegemitteln als Salze, z. B. als Alkali-, Ammonium-, Alkanolammonium- oder Aminsalze, z. B. als Salze der Aminomethylpropanols vor. Ein besonders bevorzugtes anionisches Polymer ist das Handelsprodukt Luvimer® 36 D (BASF), das als 36%ige wässrige Dispersion vorliegt .

Ein Polymeradditiv, das die Rheologie des Systems in erwünschter Weise beeinflußt und z. B. das Fädenziehen reduziert und die Filmelastizität steigert, ist ein vernetztes Copolymer des Acrylamids und des 2-Acrylamido-2-methylpropansulfonats (40 Gew.%) in Isoparaffin (20 Gew.-%) und Laureth 7 (6 Gew.-%), emulgiert in Wasser (ca. 34 Gew.-%). Ein solches Produkt ist mit der Verkaufsbeziehung Sepigel® 305 im Handel. Das Produkt ist bevorzugt in Mengen von 0,2 - 2,0 Gew.-% in dem erfindungsgemäßen Hautreinigungsmittel enthalten.

synthetische, kationische Polymere sind solche, die als Monomere oder Comonomere Dimethylaminoethylacrylat, Dimethylaminopropylacrylamid oder die entsprechenden Methacrylate und Methacrylamide, Dimethylaminostyrol, Vinylpyridin oder Methyl-diallylamin enthalten.

Andere kationische Polymere leiten sich von natürlichen Polysacchariden wie z. B. Stärke, Cellulose oder Guar ab, die durch Umsetzung z. B. mit Epoxypropyl-dimethylamin und ggf. durch anschließende Quatemierung in kationische Polymere überführt werden. Besonders eignen sich als kationische Polymere die natürlichen Chitosane (deacetyliertes Chitin), die z. B. im Handel unter dem Warenzeichen Hydagen CMF-P (Henkel KGaA) erhältlich sind und durch Salzbildung mit anorganischen oder niedermolekularen organischen Säuren in Wasser leicht löslich sind. Geeignete Säuren zur Salzbildung sind z. B. Salzsäure, Schwefelsäure, insbesondere aber Essigsäure, Glycolsäure oder Milchsäure.

In einer besonders bevorzugten Ausführung enthalten die erfindungsgemäßen Hautreinigungsmittel als ionische Polymere entweder ein Copolymer der Acryl- oder Methacrylsäure mit nichtionischen Monomeren mit einem Säureäquivalentgewicht von 300 - 500 D oder ein Chitosan, jeweils in Form eines wasserlöslichen Salzes. Als besonders gut geeignetes anionisches Polymer hat sich ein Copolymer aus Methacrylsäure, Ethylacrylat und tert. Butylacrylat mit einem K-Wert von 30 - 50 in einer Menge von 1 - 10 Gew.-% erwiesen, das mit Aminomethylpropanol neutralisiert ist. Als besonders gut geeignetes kationisches Polymer hat sich ein Chitosan in einer Menge von 0,1 - 1 Gew.% erwiesen, das mit Glycolsäure neutralisiert ist.

Als Träger für die genannten polymeren Filmbildner eignet sich Wasser mit einem Zusatz von wassermischbaren, niedrigsiedenden Alkoholen, insbesondere von Ethanol oder Isopropylalkohol. Der Alkohol dient dabei der Verkürzung der Trocknungszeit und als Lösungsmittel zur klaren Auflösung der in reinem Wasser nicht klar löslichen Polymeren.

Der pH-Wert wird auf einen Wert im Bereich von 4 - 9 eingestellt. Formulierungen, die ein anionisches Polymer enthalten, werden bevorzugt auf einen pH-Wert von 7,5 - 9 eingestellt. Zubereitungen, die ein kationisches Polymer enthalten, werden bevorzugt auf pH-Werte von 4 - 6 eingestellt.

Obwohl ein Alkoholanteil zur Verringerung der Trocknungszeit erwünscht ist, sollte sein Anteil doch nicht mehr als 30 Gew.-% des Hautreinigungsmittels betragen, da sonst leicht Hautreizungen auftreten können. In einer bevorzugten Ausführung ist als Komponente des wässrig-alkoholischen Trägers 5 - 30 Gew.-% Ethanol, Isopropanol oder ein Gemisch davon enthalten. Die besonders bevorzugte Menge an Ethanol in den erfindungsgemäßen Hautreinigungsmitteln beträgt 5 - 15 Gew.-%.

Daneben kann das erfindungsgemäße Hautreinigungsmittel weitere hautpflegende oder kosmetisch wirksame Komponenten in einer Gesamtmenge von bis zu 5 Gew.-% enthalten. Solche Komponenten sind z. B.:
- Feuchthaltemittel wie Glycerin, 1,2 - Propylenglycol, Pyrrolidoncarbonsäure, Lactate, Proteinhydrolysate, Aloe-Gel,
- Keratolytische Substanzen wie z. B. Harnstoff, Salicylsäure, Gluconsäure und andere Alpha-Hydroxycarbonsäuren oder deren Salze zur Unterstützung der Peeling-Wirkung,
- Antimikrobielle Substanzen, z. B. Zink- und Aluminiumsalze, 1,6-Hexandiol, Phenoxyethanol, p-Hydroxybenzoesäureester,
- Pflanzenextrakte wie z. B. Extrakte aus Hamamelis, Paeonien, grünem Tee, Hagebutten, Birkenblätter- und Algenextrakte,
- kosmetische Öle, z. B. Pflanzenöle, synthetische Triglyceridöle, Fettsäure- und Fettalkoholester, Paraffinöle oder Silikonöle, bevorzugt niedrigsiedende cyclische Dimethyl(poly)-siloxane. Die kosmetischen Ölkomponenten werden dabei bevorzugt in emulgierter oder mikroemulgierter Form zugesetzt,
- entzündungshemmende Wirkstoffe, wie z. B. Panthenol oder Salze der Pantothensäure, Glycyrrhizinsäure und deren Salze, Alpha-Bisabolol, Azulen oder Allantoin,
- durchblutungsfördernde Stoffe, z. B. Kastanien-Extrakte,
- Antioxidantien,
- Duftstoffe
- Pigmente. Diese dienen nicht nur der Trübung, sondern machen den Film auf der Haut sichtbar. Dadurch wird das Auffinden und die Kontrolle der restlosen Entfernung von der Haut sehr erleichtert. Ein Gehalt von 0,1 - 2 Gew.-% eines geeigneten Pigments, z. B. Titandioxid oder Eisenoxid, ist daher eine bevorzugte weitere Komponente des erfindungsgemäßen Hautpflegemittels.

Die Herstellung des Hautpflegemittels bereitet üblicherweise keine Probleme. Es empfiehlt sich aber, zunächst die polymeren Filmbildner in warmem Wasser zu lösen und dann das Ethanol zuzugeben. Ölkomponenten und lipophile Wirkstoffe werden zunächst in eine Emulsion oder Mikroemulsion überführt. Dann werden die übrigen Stoffe eingemischt. Zum Schluß wird das Parfümöl zugesetzt.

Das fertige Hautpflegemittel wird aus einer Tube mit kleiner Öffnung (von ca. 0,2 - 1 mm Durchmesser) oder aus einem Fläschchen mit einem am Deckel befestigten Pinsel auf die Haut aufgetragen. Hierzu sollte die Viskosität des Produktes nicht höher als ca. 50 Pa·s (20 °C), gemessen mit einem Brookfield Rotationsviskosimeter, sein, bevorzugt liegt die Viskosität bei etwa 10 - 30 Pa·s (20°C)

Nach einer Antrocknungszeit von ca. 5 - 15 Minuten ist der Film fest genug, um von der Haut abgezogen zu werden. Er läßt sich dann in einem einzigen Stück - oder in wenigen großen Stücken - und ohne Schmerzen von der Haut abziehen, ohne daß Reste des Films auf der Haut verbleiben, wobei Unreinheiten und Mitesser entfernt werden.

Die folgenden Beispiele sollen den Erfindungsgegenstand näher erläutern, ohne ihn hierauf zu beschränken.

### Beispiele

Folgende Hautreinigungsmittel wurden durch Vermischen der Komponenten hergestellt:

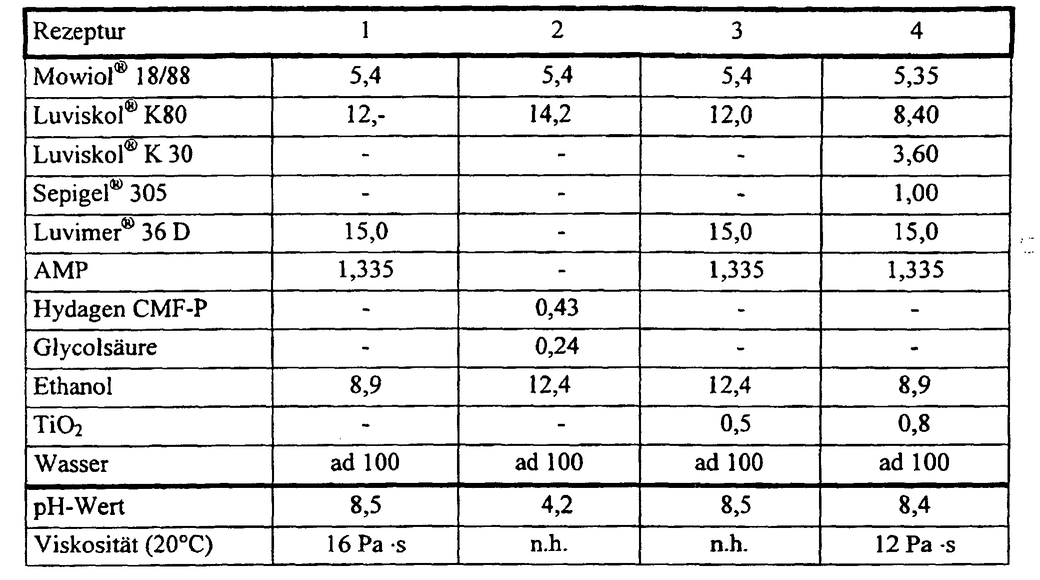

Es wurden die folgenden Handelsprodukte verwendet:
- Mowiol® 18/88:: Polyvinylalkohol (teilverseift, 12 Mol% Vinylacetat-Gruppen)
- Luviskol® K 80:: Polyvinylpyrrolidon, MG: 700 - 1000 x 10³ D
- Luviskol® K 30:: Polyvinylpyrrolidon, MG: 45 - 55 x 10³ D
- Luvimer® 36 D:: 36 %ige Dispersion von tert.-Butylacrylat/Ethylacrylat/Methacryl säure-Copolymer in Wasser, Kennzahlen (des wasserfreien Copolymers):
SZ (Säurezahl, DIN 53402): 140 - 160
K-Wert (Din 53012): 34 - 40
- Sepigel® 305:: Acylamid-/ 2-acrylamido-2-methylpropansulfanat-Copolymer (40 Gew.-%), C₁₃/C₁₄-Isoparaffin (20 Gew.-%), Laureth 7 (5 Gew.-%) Wasser (ad 100 Gew.-%)
- AMP:: Aminomethylpropanol
- Hydagen HCMF:: Chitosan

## Patentansprüche

1. Hautreinigungsmittel in Form einer fließfähigen oder auf der Haut verstreichbaren Zubereitung, die beim Antrocknen auf der Haut einen abziehbaren Film bildet, enthaltend gelöste, filmbildende und adhäsive Polymere in einem wässrig-alkoholischen Träger, **dadurch gekennzeichnet, dass** die Kombination aus den gelösten filmbildenden und adhäsiven Polymeren in einer Menge von 10 - 30 Gew.-% in dem Trägermedium enthalten ist und dass als filmbildendes Polymer ein teilacetylierter Polyvinylalkohol mit 10 - 40 Mol% Vinylacetat-Gruppen, als adhäsives Polymer ein Polyvinylpyrrolidon und weiterhin 0,1 - 10 Gew.-% eines ionischen Polymers in Form eines wasserlöslichen Salzes, ausgewählt aus
- den anionischen Homo- oder Copolymerisaten der Acrylsäure, Methacrylsäure, Crotonsäure, Itaconsäure, Maleinsäure, Fumarsäure, 2-Acrylamido-2-methylpropansulfonsäure oder Styrolsulfonsäure,
- den synthetischen kationischen Polymeren, die als Monomere oder Comonomere Dimethylaminoethylacrylat, Dimethylaminopropylacrylamid oder die entsprechenden Methacrylate und Methacrylamide, Dimethylaminostyrol, Vinylpyridin oder Methyldiallylamin enthalten,
- den von natürlichen Polysacchariden wie z. B. Stärke, Cellulose oder Guar abgeleiteten kationischen Polymeren
- den Chitosanen und
- zwitterionischen Polymeren.
enthalten ist

2. Hautreinigungsmittel nach Anspruch 1, **dadurch gekennzeichnet, dass** das ionische Polymer ausgewählt ist aus den Copolymeren der Acryl- oder Methacrylsäure mit einem Säureäquivalentgewicht von 300 - 500 D.

3. Hautreinigungsmittel nach Anspruch 2, **dadurch gekennzeichnet, dass** das Acryl- oder Methacrylsäure-Copolymer einen K-Wert von 30 - 50 hat und in einer Menge von 1 - 10 Gew.-% enthalten ist.

4. Hautreinigungsmittel nach Anspruch 1, **dadurch gekennzeichnet, dass** die anionischen Copolymerisate ausgewählt sind aus Copolymeren der Acryl- oder Methacrylsäure mit nichtionischen Monomeren aus der Gruppe der Acrylsäure-(C₁-C₄)-alkylester sowie aus einem vernetzten Copolymer des Acrylamids und des 2-Acrylamido-2-methylpropansulfonats (40 Gew.-%) in Isoparaffin (20 Gew.-%) und Laureth 7 (6 Gew.-%), emulgiert in Wasser (ca. 34 Gew.-%).

5. Hautreinigungsmittel nach einem der Ansprüche 1 - 4, **dadurch gekennzeichnet, dass** der teilacetylierte Polyvinylalkohol in einer Menge von 2 - 10 Gew.-% enthalten ist.

6. Hautreinigungsmittel nach einem der Ansprüche 1 - 5, **dadurch gekennzeichnet, dass** ein Polyvinylpyrrolidon oder ein Gemisch von Polyvinylpyrrolidonen unterschiedlicher Molekulargewichte mit einem mittleren Molekulargewicht von wenigstens 40.000 D, bevorzugt von 700.000 - 1.000.000 D, in einer Menge von 1 - 20 Gew.-% enthalten ist.

7. Hautreinigungsmittel nach einem der Ansprüche 1 - 6 **dadurch gekennzeichnet, dass** auch andere nichtionische, wasserlösliche Polymere, wie z. B. Polyacrylamide oder Polyethylenglycole, in Mengen bis 5 Gew.-% enthalten sind.

8. Hautreinigungsmittel nach einem der Ansprüche 1 - 7, **dadurch gekennzeichnet, dass** als Komponente des wässrig-alkoholischen Trägers 5 - 30 Gew.-% des Hautreinigungsmittels Ethanol, Isopropanol oder ein Gemisch davon enthalten ist.

9. Hautreinigungsmittel nach einem der Ansprüche 1 - 8, **dadurch gekennzeichnet, dass** der pH-Wert auf einen Wert im Bereich von 4 - 9 eingestellt wird.

10. Hautreinigungsmittel nach einem der Ansprüche 1 - 9, **dadurch gekennzeichnet, dass** weitere hautpflegende oder kosmetisch wirksame Komponenten in einer Gesamtmenge von bis zu 5 Gew.-% enthalten sind, ausgewählt aus
- Feuchthaltemitteln,
- keratolytischen Substanzen,
- antimikrobiellen Substanzen,
- Pflanzenextrakten,
- kosmetischen Ölen,
- entzündungshemmenden Wirkstoffen,
- durchblutungsfördernden Stoffen,
- Antioxidantien,
- Duftstoffen und
- Pigmenten.

11. Hautreinigungsmittel nach einem der Ansprüche 1 - 10, **gekennzeichnet durch** eine Viskosität von 10 *-* 50 Pa·s (20°C), gemessen mit einem Brookfield Rotationsviskosimeter.

12. Hautreinigungsmittel nach einem der Ansprüche 1 - 11, **dadurch gekennzeichnet, dass** es aus einer Tube mit einer Öffnung von 0,2 - 1 mm Durchmesser oder aus einem Fläschchen mit einem am Deckel befestigten Pinsel oder mit einem Pinsel oder mit einem Rollkugelapplikator oder mit dem Finger auf die Haut aufgetragen wird.

## Revendications

1. Produit de nettoyage de la peau sous la forme d'une préparation fluide ou applicable sur la peau par étalement, qui, en séchant sur la peau, forme une pellicule pelable, contenant des polymères dissous, filmogènes et adhésifs dans un support aqueux-alcoolique, **caractérisé en ce que** le milieu de support contient la combinaison des polymères dissous, filmogènes et adhésifs en une proportion de 10 - 30 % en poids et **en ce que**, comme polymère filmogène, il comprend un alcool polyvinylique partiellement acétylé contenant 10 - 40 % en moles de groupes acétate de vinyle, comme polymère adhésif, il comprend une polyvinylpyrrolidone et en outre, il comprend 0,1-10 **%** en poids d'un polymère ionique sous la forme d'un sel hydrosoluble, choisi parmi
- les homo- ou copolymères anioniques de l'acide acrylique, méthacrylique, crotonique, itaconique, maléique, fumarique, 2-acrylamido-2-méthyl-propanesulfonique ou styrènesulfonique,
- les polymères cationiques synthétiques contenant, en tant que monomère ou comonomère, l'acrylate de diméthylaminoéthyle, le diméthylaminopropylacrylamide ou les méthacrylates et les méthacrylamides correspondants, le diméthylaminostyrène, la vinylpyridine ou la méthyldiallylamine,
- les polymères cationiques dérivant de polysaccharides naturels tels que, par exemple, l'amidon, la cellulose ou la gomme guar,
- les chitosanes, et
- les polymères zwitterioniques.

2. Produit de nettoyage de la peau selon la revendication 1, **caractérisé en ce que** le polymère ionique est choisi parmi les copolymères d'acide acrylique ou méthacrylique ayant un poids équivalent d'acide de 300 - 500 D.

3. Produit de nettoyage de la peau selon la revendication 2, **caractérisé en ce que** les copolymères d'acide acrylique ou méthacrylique présentent une valeur de K de 30 - 50 et sont contenus dans une proportion de 1 - 10 % en poids.

4. Produit de nettoyage de la peau selon la revendication 1, **caractérisé en ce que** les copolymères anioniques sont choisis parmi les copolymères d'acide acrylique ou méthacrylique formés avec des monomères non ioniques du groupe des esters alkyle en C₁-C₄ de l'acide acrylique ainsi que d'un copolymère réticulé de l'acrylamide et du 2-acrylamido-2-méthylpropanesulfonate (40 % en poids) dans de l'isoparaffine (20 % en poids) et du Laureth 7 (6 % en poids), émulsifié dans de l'eau (environ 34 % en poids).

5. Produit de nettoyage de la peau selon l'une quelconque des revendications 1 - 4, **caractérisé en ce qu'**il contient l'alcool polyvinylique partiellement acétylé dans une proportion de 2 -10 % en poids.

6. Produit de nettoyage de la peau selon l'une quelconque des revendications 1 - 5, **caractérisé en ce qu'**il contient une polyvinylpyrrolidone ou un mélange de polyvinylpyrrolidones de différents poids moléculaires, ayant un poids moléculaire moyen d'au moins 40 000 D, de préférence de 700 000-1 000 000 D, dans une proportion de 1-20 % en poids.

7. Produit de nettoyage de la peau selon l'une quelconque des revendications 1 - 6, **caractérisé en ce qu'**il contient également d'autres polymères hydrosolubles non ioniques, tels que, par exemple, des polyacrylamides ou des polyéthylèneglycols, dans une proportion allant jusqu'à 5 % en poids.

8. Produit de nettoyage de la peau selon l'une quelconque des revendications 1 - 7, **caractérisé en ce que**, en tant que composant du support aqueux-alcoolique, il contient 5 - 30 % en poids, par rapport au produit de nettoyage de la peaux, d'éthanol, d'isopropanol ou d'un mélange de ces composants.

9. Produit de nettoyage de la peau selon l'une quelconque des revendications 1 - 8, **caractérisé en ce que** le pH est ajusté à une valeur comprise dans la gamme de 4 - 9.

10. Produit de nettoyage de la peau selon l'une quelconque des revendications 1 - 9, **caractérisé en ce qu'**il contient en outre d'autres composants de type produit de soin de la peau ou produit cosmétique dans une proportion allant jusqu'à 5 % en poids, choisis parmi les
- humidifiants,
- substances kératolytiques,
- substances anti-microbiennes,
- extraits de plantes,
- huiles cosmétiques,
- principes actifs anti-inflammatoires,
- substances favorisant la vascularisation,
- antioxydants,
- parfums, et
- pigments.

11. Produit de nettoyage de la peau selon l'une quelconque des revendications 1 - 10, **caractérisé par** une viscosité de 10 - 50 Pa^{·}s (20°C), déterminée à l'aide d'un viscosimètre à rotation Brookfield.

12. Produit de nettoyage de la peau selon l'une quelconque des revendications 1 - 11, **caractérisé en ce que** qu'il est appliqué sur la peau à partir d'un tube ayant un orifice de 0,2 - 1 mm de diamètre ou d'un flacon muni d'un pinceau fixé sur le couvercle ou à l'aide d'un pinceau ou d'un applicateur à bille roulante, ou avec le doigt.

## Claims

1. A skin cleansing composition in the form of a flowable preparation or one which can be spread over the skin and which, on drying, forms a peelable film on the skin, containing dissolved, film-forming and adhesive polymers in a water/alcohol carrier, **characterized in that** the combination of the dissolved film-forming and adhesive polymers is present in a quantity of 10 to 30% by weight in the carrier medium and **in that** the film-forming polymer is a partly acetylated polyvinyl alcohol containing 10 to 40 mol-% vinyl acetate groups, the adhesive polymer is a polyvinyl pyrrolidone and 0.1 to 10% by weight of an ionic polymer is additionally present in the form of a water-soluble salt selected from
- the anionic homo- or copolymers of acrylic acid, methacrylic acid, crotonic acid, itaconic acid, maleic acid, fumaric acid, 2-acrylamido-2-methylpropanesulfonic acid or styrene sulfonic acid,
- the synthetic cationic polymers which contain dimethyl aminoethyl acrylate, dimethyl aminopropyl acrylamide or the corresponding methacrylates and methacrylamides, dimethyl aminostyrene, vinyl pyridine or methyl diallylamine as monomers or comonomers,
- the cationic polymers derived from natural polysaccharides such as, for example, starch, cellulose or guar,
- the chitosans and
- zwitterionic polymers.

2. A skin cleansing composition as claimed in claim 1, **characterized in that** the ionic polymer is selected from the copolymers of acrylic or methacrylic acid with an acid equivalent weight of 300 to 500 D.

3. A skin cleansing composition as claimed in claim 2, **characterized in that** the acrylic or methacrylic acid copolymer has a K value of 30 to 50 and is present in a quantity of 1 to 10% by weight.

4. A skin cleansing composition as claimed in claim 1, **characterized in that** the anionic copolymers are selected from copolymers of acrylic or methacrylic acid with nonionic monomers from the group of acrylic acid (C₁₋₄) alkyl esters and from a crosslinked copolymer of acrylamide and 2-acrylamido-2-methylpropanesulfonate (40% by weight) in isoparaffin (20% by weight) and Laureth 7 (6% by weight) emulsified in water (ca. 34% by weight).

5. A skin cleansing composition as claimed in any of claims 1 to 4, **characterized in that** the partly acetylated polyvinyl alcohol is present in a quantity of 2 to 10% by weight.

6. A skin cleansing composition as claimed in any of claims 1 to 5, **characterized in that** a polyvinyl pyrrolidone or a mixture of polyvinyl pyrrolidones differing in their molecular weights with an average molecular weight of at least 40,000 D and preferably in the range from 700,000 to 1,000,000 D is present in a quantity of 1 to 20% by weight.

7. A skin cleansing composition as claimed in any of claims 1 to 6, **characterized in that** other nonionic water-soluble polymers, such as polyacrylamides or polyethylene glycols for example, are also present in quantities of up to 5% by weight.

8. A skin cleansing composition as claimed in any of claims 1 to 7, **characterized in that** 5 to 30% by weight of the skin cleaning composition of ethanol, isopropanol or a mixture thereof is present as a component of the water/alcohol carrier.

9. A skin cleansing composition as claimed in any of claims 1 to 8, **characterized in that** the pH is adjusted to a value of 4 to 9.

10. A skin cleansing composition as claimed in any of claims 1 to 9, **characterized in that** other skin-care or cosmetically active components selected from
- moisturizers,
- keratolytic substances,
- antimicrobial agents,
- plant extracts,
- cosmetic oils,
- anti-inflammatory agents,
- circulation-stimulating agents,
- antioxidants,
- perfumes and
- pigments
are present in a total quantity of up to 5% by weight.

11. A skin cleansing composition as claimed in any of claims 1 to 10, **characterized by** a viscosity of 10 to 50 Pa.s (20°C), as measured with a Brookfield rotational viscosimeter.

12. A skin cleansing composition as claimed in any of claims 1 to 11, **characterized in that** it is applied to the skin from a tube with an opening between 0.2 and 1 mm in diameter or from a bottle with a brush attached to the cover or by a brush or by a roller ball applicator or by a finger.
